# EUROPEAN PATENT APPLICATION

(11) **EP 2 270 496 A1**
(43) Date of publication of application: **05.01.2011**
(21) Application number: 09163931.0
(22) Date of filing: 26.06.2009
(51) Int. Cl.: G01N 33/497

(54) **Method for the diagnosis of cystic fibrosis by detecting volatile organic compounds in exhaled air.**

(71) Applicant: Universiteit Maastricht, 6211 LK Maastricht (NL); Academisch Ziekenhuis Maastricht, 6229 HX Maastricht (NL)
(72) Inventor: Dallinga, Jan Willem, 6411 VA Heerlen (NL); Van Schooten, Frederik Jan, 6247 BB Gronsveld (NL); Van Berkel, Joep Joseph Benjamin Nathan, 6224 CS Maastricht (NL)
(74) Representative: Habets, Winand

(57) **Abstract**

The present invention relates to the identification of markers for the disease conditions related to cystic fibrosis (CF). The uses of such markers in diagnosis and a novel method for their identification are herein described. We were able to discriminate between patients with cystic fibrosis and normal individuals by determining whether the exhaled air contained markers selected from that set. We also found that these markers can be used in the early diagnosis of cystic fibrosis. Moreover, these markers are useful in the prediction of the occurrence of cystic fibrosis even when no other clinical signs are apparent yet.
In particular, the marker is selected from the group consisting of 3,3-dimethylhex-1-ene, 2-buten-1-ol, N-methyl-2-methylpropylamine, C8H16 hydrocarbon (2-octene, 3-octene), tolualdehyde (o-, m-, or p- isomers), C16 poly-unsaturated hydrocarbon, C12 saturated hydrocarbon, C13 saturated hydrocarbon, benzothiazole and long chain alkylbenzenes.

## Description

### Field of the invention

The present invention relates to the identification of markers for the disease conditions related to cystic fibrosis (CF). The uses of such markers in diagnosis and a novel method for their identification are disclosed herein.

### Background of the invention

Cystic fibrosis (also known as CF, mucovoidosis, or mucoviscidosis) is a genetic disorder known to be an inherited disease of the secretory glands, including the glands that make mucus and sweat.

The hallmarks of cystic fibrosis are salty tasting skin, appetite but poor growth and poor weight gain, excess mucus production, and coughing/shortness of breath. Males can be infertile due to the condition congenital bilateral absence of the vas deferens. Often, symptoms of CF appear in infancy and childhood. Meconium ileus is a typical finding in newborn babies with CF.

Although technically a rare disease, cystic fibrosis is ranked as one of the most widespread life-shortening genetic diseases. It is most common among nations in the Western world; one in twenty-two people of Mediterranean descent is a carrier of one gene for CF, making it the most common genetic disease in these populations. An exception is Finland, where only one in 80 people carry a CF mutation.[Hytönen M, et al., Acta Oto-laryngologica 2001, 121 (8): 945-7] In the United States, 1 in 4,000 children are born with CF. In 1997, about 1 in 3,300 caucasian children in the United States was born with cystic fibrosis. In contrast, only 1 in 15,000 African American children suffered from cystic fibrosis, and in Asian Americans the rate was even lower at 1 in 32,000.

Cystic fibrosis occurs when there is a mutation in the CFTR gene. The protein created by this gene is anchored to the outer membrane of cells in the sweat glands, lungs, pancreas, and other affected organs. The protein spans this membrane and acts as a channel connecting the inner part of the cell (cytoplasm) to the surrounding fluid. This channel is primarily responsible for controlling the movement of chloride from inside to outside of the cell; however, in the sweat ducts it facilitates the movement of chloride from the sweat into the cytoplasm. When the CFTR protein does not work, chloride is trapped inside the cells in the airway and outside in the skin. Because chloride is negatively charged, positively charged ions cross into the cell because they are affected by the electrical attraction of the chloride ions. Sodium is the most common ion in the extracellular space and the combination of sodium and chloride creates the salt, which is lost in high amounts in the sweat of individuals with CF. This lost salt forms the basis for the sweat test.[Rowe SM et al., N Engl J Med. 2005; 352(19):1992-2001]

How this malfunction of cells in cystic fibrosis causes the clinical manifestations of CF is not well understood. One theory suggests that the lack of chloride exodus through the CFTR protein leads to the accumulation of more viscous, nutrient-rich mucus in the lungs that allows bacteria to hide from the body's immune system. Another theory proposes that the CFTR protein failure leads to a paradoxical increase in sodium and chloride uptake, which, by leading to increased water reabsorption, creates dehydrated and thick mucus. Yet another theory focuses on abnormal chloride movement out of the cell, which also leads to dehydration of mucus, pancreatic secretions, biliary secretions, etc. These theories all support the observation that the majority of the damage in CF is due to blockage of the narrow passages of affected organs with thickened secretions. These blockages lead to remodeling and infection in the lung, damage by accumulated digestive enzymes in the pancreas, blockage of the intestines by thick faeces, etc.[Rowe SM et al., N Engl J Med. 2005; 352(19):1992-2001]

Cystic fibrosis may be diagnosed by many different categories of testing including those such as, newborn screening, sweat testing, or genetic testing. As of 2006 in the United States, 10 percent of cases are diagnosed shortly after birth as part of newborn screening programs. The newborn screen initially measures for raised blood concentration of immunoreactive trypsinogen.[Davies J et al. BMJ. 2007 335(7632):1255-59] Infants with an abnormal newborn screen need a sweat test in order to confirm the CF diagnosis. Trypsinogen levels can be increased in individuals who have a single mutated copy of the CFTR gene (carriers) or, in rare instances, even in individuals with two normal copies of the CFTR gene. Due to these false positives, CF screening in newborns is somewhat controversial.

Most states and countries do not screen for CF routinely at birth. Therefore, most individuals are diagnosed after symptoms that prompt an evaluation for cystic fibrosis. The most commonly used form of testing is the sweat test. Sweat-testing involves application of a medication that stimulates sweating (pilocarpine) to one electrode of an apparatus and running electric current to a separate electrode on the skin. This process, called iontophoresis, causes sweating; the sweat is then collected on filter paper or in a capillary tube and analyzed for abnormal amounts of sodium and chloride. People with CF have increased amounts of sodium and chloride in their sweat. CF can also be diagnosed by identification of mutations in the CFTR gene.[Stern, RC. N Engl J Med 1997; 336:487]

A multitude of tests are used to identify complications of CF and to monitor disease progression. X-rays and CAT scans are used to examine the lungs for signs of damage or infection. Examination of the sputum under a microscope is used to identify which bacteria are causing infection so that effective antibiotics can be given. Pulmonary function tests measure how well the lungs are functioning, and are used to measure the need for and response to antibiotic therapy. Blood tests can identify liver abnormalities, vitamin deficiencies, and the onset of diabetes. DEXA scans can screen for osteoporosis and testing for fecal elastase can help diagnose insufficient digestive enzymes.

Because not all known mutations are found on current tests, a negative screen does not guarantee that a person will not have CF [Tabor A, Philip J, et al., Lancet. 1986, 1(8493):1287-93]. During pregnancy, testing can be performed on the placenta (chorionic villus sampling) or the fluid around the fetus (amniocentesis). However, chorionic villus sampling has a risk of fetal death of 1 in 100 and amniocentesis of 1 in 200 [Tabor A, Philip J, et al., Lancet. 1986, 1 (8493):1287-93] although a recent study has indicated this may actually be much lower, perhaps 1 in 1,600 [ Hytönen M, et al., Acta Oto-laryngologica 2001, 121 (8): 945-7] so the benefits must be determined to outweigh these risks prior to going forward with testing.

Airway inflammation plays a central role in the pathophysiology of cystic fibrosis. Diagnosing CF is possible at any age, but it is far more difficult to diagnose early pulmonary disease in CF patients, particularly in young children. It is known there is a poor correlation between airway inflammatory processes and respiratory symptoms [Gibson RL, et al., Am J Respir Crit Care Med 2003; 168:918-951]. Airway infection and inflammation can be present before clinical symptoms are evident. Thus monitoring of airway inflammation or oxidative stress in cystic fibrosis may be useful in clinical practice.

There has been an increasing interest in non-invasive assessment of airway inflammation and oxidative stress in chronic lung disease. The collection of broncho-alveolar lavage fluid or lung biopsies is invasive, and therefore cannot be applied very easily in children. Non-invasive techniques include measurement of non-volatile inflammatory markers in exhaled breath condensate, and measurement of volatile inflammatory markers in exhaled breath.

Fractional exhaled nitric oxide (FeNO), carbon monoxide (CO), ethane and pentane are the most studied volatile markers [Paredi P, et al., Am J Respir Crit Care Med 2000; 162:1450-1454. and Horvath I, et al. Eur Respir J 2001; 18:420-430,] of which FeNO is most standardised [ATS/ERS Recommendations for Standardized Procedures for the Online and Offline Measurement of Exhaled Lower Respiratory Nitric Oxide and Nasal Nitric Oxide, 2005. Am J Respir Crit Care Med 2005; 171:912-930]. In 2006, Barker et al. studied 12 specific VOCs by means of a customized gas chromatograph in CF patients and controls. They reported a significant lower level of dimethyl sulphide (DMS) in CF compared to controls [Barker M, et al. Eur Respir J 2006; 27:929-936] However, in this study only allows conclusions on a group level; no individual classification of subjects was performed.

There remains a need in the art for more and better diagnostic markers for cystic fibrosis, in particular for markers that can be used in a non-invasive diagnostic method. The present invention addresses those needs.

### Summary of the invention

We analyzed the volatile organic compounds (VOCs) in exhaled air of patients with cystic fibrosis by using a gas chromatograph - time of flight - mass spectrometer (GC-TOF-MS). We found a set of markers that may be used for the diagnosis of cystic fibrosis.

We were able to discriminate between patients with cystic fibrosis and normal individuals by determining whether the exhaled air contained markers selected from that set.

More in particular we found that these markers can be used in the early diagnosis of cystic fibrosis. Moreover, these markers are useful in the prediction of the occurrence of cystic fibrosis even when no other clinical signs are apparent yet.

The method is also useful since it provides a new non-invasive, easy, safe, cost effective and fast diagnostic tool for the diagnosis of cystic fibrosis.

Hence, the invention relates to an in vitro method of diagnosing cystic fibrosis in a patient by determining the amount of at least one volatile organic compound in a sample derived from a patient suspected of having cystic fibrosis and comparing said amount with a normal value for said volatile organic compound, wherein a difference between the normal value and the amount of said at least one volatile organic compound is indicative of cystic fibrosis, wherein said volatile organic compound is selected from the group consisting of 3,3-dimethylhex-1-ene, 2-buten-1-ol, N-methyl-2-methylpropylamine, C8H16 hydrocarbon (2-octene, 3-octene), tolualdehyde (o-, m-, or p- isomers), C16 polyunsaturated hydrocarbon, C12 saturated hydrocarbon, C13 saturated hydrocarbon, benzothiazole and long chain alkylbenzenes.

### Detailed description of the invention

An increased production of reactive oxygen species (ROS) may be caused by the influx of leukocytes, which continuously produce ROS, leading to an imbalance between oxidants and antioxidants (oxidative stress). ROS can degrade cell membranes by lipid peroxidation. During this process, volatile organic compounds (VOCs) may be formed as a result of the degradation of polyunsaturated fatty acids. Endogenous VOCs can be divided in hydrocarbons, oxygen or sulphur containing compounds, and nitrogen containing substances [Miekisch W, et al., Clin Chim Acta 2004; 347:25-39].

It was possible to assess distinctive VOC profiles, which discriminated between CF patients and controls. Based on 10 times cross validation, a correct classification of CF patients and controls was found using a VOC selected from table 4.

**TABLE 4**

| *Identification of 10 most discriminating attributes between CF and controls* | | |
|---|---|---|
| **VOC No:** | **Identified as** | **Chemical formula** |
| 1 | 3,3-dimethylhex-1-ene | C₈H₁₆ |
| 2 | 2-buten-1-ol | C₄H₈O |
| 3 | N-methyl-2-methylpropylamine | C₅H₁₃N |
| 4 | C8H16 hydrocarbon (2-octene, 3-octene) | C₈H₁₆ |
| 5 | Tolualdehyde (o-, m-, or p- isomers) | C₈H₈O |
| 6 | C16 poly-unsaturated hydrocarbon | C₁₆ |
| 7 | C12 saturated hydrocarbon | C₁₂H₂₆ |
| 8 | C13 saturated hydrocarbon | C₁₃H₂₈ |
| 9 | Benzothiazole | C₇H₅NS |
| 10 | Long chain alkylbenzene | C₈-C₁₆ |

Hence, the invention relates to an in vitro method of diagnosing cystic fibrosis in a patient by determining the amount of at least one volatile organic compound in a sample derived from a patient suspected of having cystic fibrosis and comparing said amount with a normal value for said volatile organic compound, wherein a difference between the normal value and the amount of said at least one volatile organic compound is indicative of cystic fibrosis, wherein said volatile organic compound is selected from the group consisting of 3,3-dimethylhex-1-ene, 2-buten-1-ol, N-methyl-2-methylpropylamine, C8H16 hydrocarbon (2-octene, 3-octene), tolualdehyde (o-, m-, or p- isomers), C16 polyunsaturated hydrocarbon, C12 saturated hydrocarbon, C13 saturated hydrocarbon, benzothiazole and long chain alkylbenzenes.

Said sample derived from a patient is preferably a sample of exhaled air from the patient, it may also be a sample wherein the volatile organic compounds in the exhaled air are collected and/or immobilized, such as an active carbon solid phase or an affinity matrix.

A method according to the invention makes a contribution to the art as a whole, in that it provides a method with a sensitivity of more than 58% while the specificity is more than 90%. The prior art methods for diagnosing cystic fibrosis that measure volatile organic compounds in exhaled air do not provide such high specificity and/or sensitivity.

Methods to detect VOCs in exhaled air as such are known in the art. The examples provide guidance for the skilled person to perform an analysis according to the invention. Normal values may be obtained by determining VOCs in the exhaled air of normal individuals. VOCs may be detected in several ways known in the art. This may be done by a device capable of measuring more than one VOC at the same time such as a mass spectrometer or by a device specifically suitable for measuring only one VOC at a time.

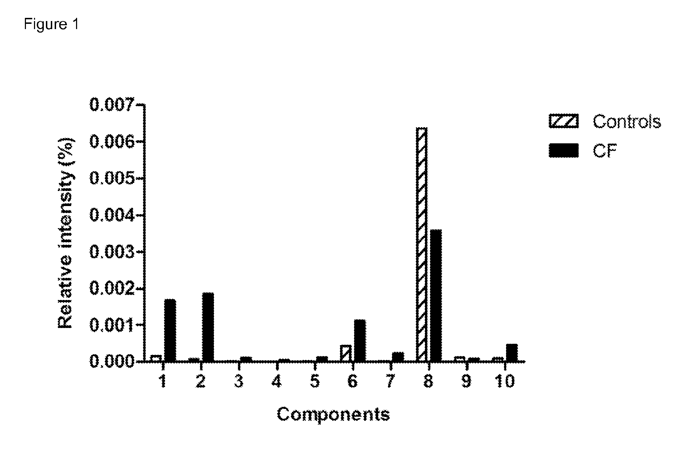

Figure 1 shows the mean relative intensity of the ten VOCs from table 4. It can be seen that VOCs 1 to 7 and 9 and 10 are increased in the population of CF patients, whereas VOC number 8 is decreased in the population of CF patients when compared to normal controls.

When a single VOC is to be used, VOC No 1, i.e. 3,3 dimethylhex-1-ene is preferred. This may be advantageous in an assay capable of detecting only a single VOC at a time such as an electronic nose.

The sensitivity, specificity, and the percentage of correct classification increased with increasing number of VOCs selected from table 4. Hence, the invention also relates to a method as described above wherein at least two volatile organic compounds are detected, such as three, four, five or six VOCs. The method may even be further improved by determining the relative amounts of seven, eight, nine VOCs. Most preferred is a method as described above wherein all ten VOCs from table 4 are determined in a method according to the invention.

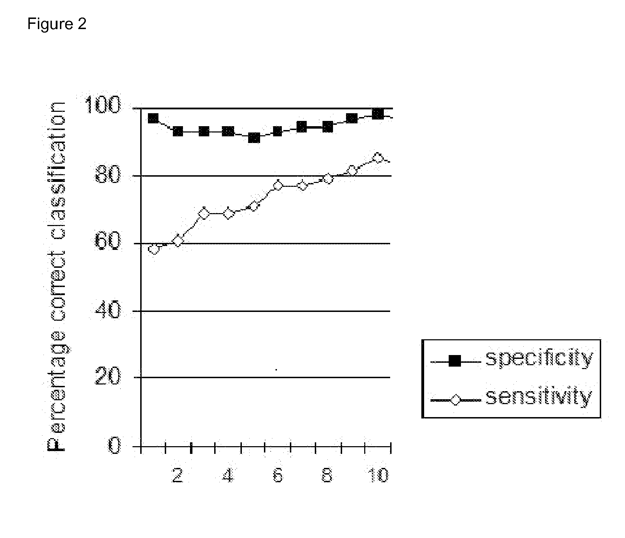

Figure 2 shows the percentage of correct classification of CF with increased numbers of VOCs. It can be seen that the percentage of correct classification increases when more than one VOC selected from table 4 is used in a method according to the invention. The highest sensitivity and specificity could be obtained when a method according to the invention was performed with all 10 VOCs from table 4 combined.

A preferred embodiment of the method according to the invention comprises the use of VOC No 1 together with a VOC selected from the group consisting VOC No 2 to VOC No. 10, preferably No 2.

### Legend to the figures

Figure 1: Mean relative intensity of the ten VOCs that are used to generate the discriminant function that discriminates CF patients from healthy controls. The numbers correspond to the VOCs in table 4.
Figure 2: . The percentage of correct classification of cystic fibrosis with increasing numbers of VOCs, using discriminant analysis. The discriminant analysis was performed using 10 times cross-validation. A VOC was only included in the analysis if its prevalence was 7% or more within the study population. The sensitivity of the method ranged from 58% to nearly 100%. The specificity of the method ranged from 91 to nearly 100%.
Figure 3: An acquired chromatogram of volatile organic compounds. The x-axis represents the gas chromatograph retention time (minutes). The y-axis shows the relative abundance of various compound signals. The cumulative area under all peaks used for classification analyses, is by definition 100%.

### Examples

### Example 1: Subjects

105 Subjects aged 5-25 years were included in this cross-sectional study: 48 subjects with a CF diagnosis, and 57 control persons. The CF population was recruited from the outpatient clinic of the University Hospital Maastricht, the Netherlands. CF was defined as a combination of typical clinical features, and an abnormal sweat test (Chloride > 60 mmol/L). Children with CF and/or their parents completed the Shwachman-Kulczycki questionnaire, enabling classification of the CF disease status [Shwachman H,and Kulczycki LL. JAMA Dis Child 1958; 96:6-15]. The control group consisted of children without any (history of) respiratory problems, as confirmed by the ISAAC questionnaire [Variations in the prevalence of respiratory symptoms, self-reported asthma attacks, and use of asthma medication in the European Community Respiratory Health Survey (ECRHS). Eur Respir J 1996; 9:687-695]. Thirty six (63%) control subjects were recruited from primary schools, whereas 21 (37%) subjects were included at the outpatient clinic where enuresis nocturna and constipation were the initial reasons for consultation. At the moment of this study, these children were all stable with no signs of infection or any somatic disturbance. We checked the homogeneity of the control group by comparing children recruited from primary schools and outpatient clinic and found no significant differences in VOC patterns between these children (One-way ANOVA tests, p-values > 0.108). Exclusion criteria for both CF and control children were: 1) Patients with mental retardation; 2) Active smokers; 3) (Congenital) heart disease; 4) Technical inability to perform the measurements; 5) Patients with an acute respiratory infection.

Informed consent to participate was obtained from the parents of all children participating in this study. The Ethics Committee of the Maastricht University approved this study. The clinical trial registration number is NCT 00413140.

Subjects with CF were characterized by a light to moderate airway obstruction, air-trapping, and a mild restrictive impairment in comparison with controls (Table 1 and 2). In the CF group, 49% of subjects had positive cultures with Pseudomonas aeruginosa in the past 2 years, and 8 persons (17%) showed a history of allergic bronchopulmonary aspergillosis (ABPA). The mean ± SE time between sputum collection and breath sample collection was 50 ± 62 days. Patients were mainly treated with antibiotics, antacids, DNase, and corticosteroids (Table 2).

**TABLE 1**

| *Clinical characteristics of the study populations* | | | | |
|---|---|---|---|---|
| | | **Cystic Fibrosis** | **Control** | **Significance*** |
| | | N = 48 | N = 57 | |
| Age | yrs | 13.0 ± 0.6 | 9.9 ± 0.4 | *P* < .05 |
| Height | cm | 148.3 ± 2.9 | 141.5 ± 2.3 | |
| Weight | kg | 38.6 ± 2.1 | 34.8 ±1.5 | |
| Sex | M / F | 27/21 | 29/28 | |
| FEV₁ % predicted | | 75.5 ± 3.8 | 101.4 ± 1.5 *P* < .05 | |
| FEV₁/VC% | | 76.7 ± 1.8 | 88.1 ± 0.9 | *P* < .05 |

| | | | | |
|---|---|---|---|---|
| Abbreviations: M, male; F, female; FEV₁, forced expiratory volume in 1 second; VC, vital capacity; Data are given as mean ± standard error (SE); * significance of cystic fibrosis versus control | | | | |

**TABLE 2**

| *Additional characteristics of the cystic fibrosis population (n=48)* | |
|---|---|
| **ALLERGY** * | **17 (36%)** |
| - Total IgE (KU/L) | 625 ± 263 |
| - Active eczema | 1 (2%) |
| - Allergic rhinitis | 2 ( 4%) |

| **TREATMENT** | |
|---|---|
| - Oral steroid | 4 ( 8%) |
| - Inhaled steroid | 7 (15%) |
| - DNase | 25 (52%) |
| - Antacid | 24 (50%) |
| - Antibiotic | 30 (63%) |

| **MICRO-ORGANISM COLONIZATION** | **40 (83%)** |
|---|---|
| - Pseudomonas aeruginosa | 23 (48%) |
| - Staphylococcus Aureus | 29 (60%) |
| - Haemophilus Influenzae | 14(29%) |
| - Haemophilus Parainfluenzae | 3 (6%) |
| - Aspergillus Fumigatus | 17 (35%) |
| - Candida Albicans | 6 (13%) |

| **LUNG FUNCTION INDICES** | |
|---|---|
| - Reversibility § | 19% |
| - TLC % predicted | 95.2 ± 4.8 |
| - RV % predicted | 182.4 ± 18.8 |
| - ITGV % predicted | 114.7 ± 7.3 |

| | |
|---|---|
| Abbreviations: ITGV, intra-thoracic gas volume; RV, residual volume; TLC, total lung capacity. Data are given as mean ± SE except were indicated otherwise. * A child was considered allergic when the total IgE level exceeded 20 kU/I and / or the Phadiatop was positive and / or the Radio Allergo Sorbent Test (RAST) had two or more allergens ≥ class 2. § Reversibility is defined as the presence of an increase in FEV1 of 9% of predicted value or more after inhalation of 400 µg salbutamol [Dundas I, et al. Thorax 2005; 60:13-16.] | |

### Example 2: Exhaled breath analysis

Subjects were asked to exhale into a resistance free polycarbonate (plastic) bag (Tedlar bag, SKC ltd., Dorset, UK). Severe physical exercise before the test was not permitted. At least three exhalations were necessary to fill the 5-liter bag. Within one hour after collection of the sample, the bag was emptied over a stainless steel two-bed sorption tube, filled with carbograph 1TD/Carbopack X (Markes International, Llantrisant, Wales, UK). Before and after loading, the tubes were airtight capped. The desorption tubes were stored at room temperature until analysis. All subjects completed the maneuver correctly. No adverse effects were reported during the exhaled breath collection.

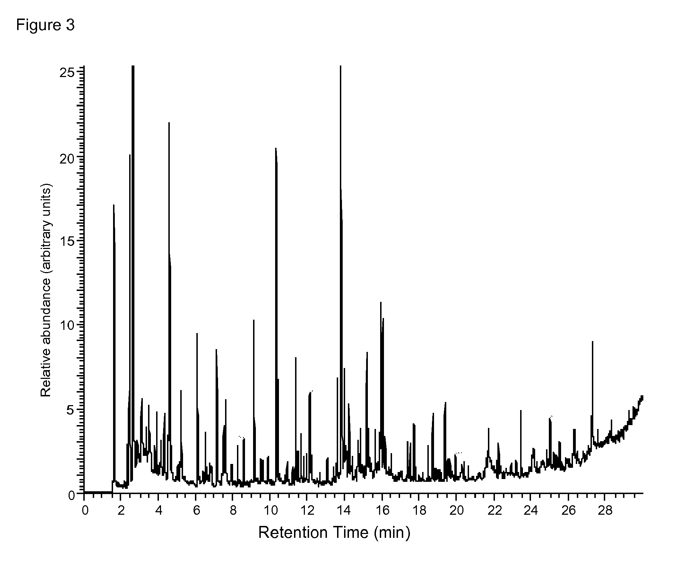

Analysis of the samples started with releasing the volatile compounds trapped on the sorption tubes by thermal desorption, using the Markes International Ultra-Unity automated thermal desorption equipment (Markes International, Llantrisant, Wales, UK). The gaseous mixture of released compounds was then split; 90% of the sample was recollected on a second sorption tube and stored for a possible second analysis, 10% of the sample was loaded onto a cold (5 °C) sorption trap, from which it was injected into a gas chromatograph (Trace GC, ThermoFischer Scientific, Austin, Texas, USA) and analyzed by time-of-flight mass spectrometry (Tempus Plus, ThermoFischer Scientific, Austin, Texas, USA). For the GC-MS measurements the following conditions were used: column: Restek RTX-5ms, 30m x 0.25 mm ID, coated with 1.0 um HP-5 phase; helium was used as the carrier gas at a flow rate of 1.5 ml / min. The temperature of the gas chromatograph was programmed as follows: 40 °C during 5 min., then raised with 10 °C/min until a final maximum temperature of 270 °C. In the final step this temperature was maintained for 5 min. The mass spectrometer was set at a scan range of 35-350 amu and scanned at 5Hz. A representative example of a chromatogram is shown in figure 3.

The GC-MS chromatograms of the breath samples of the 105 subjects involved in this research were recorded and corrected for retention time differences, using retention indices (relative to toluene), and lining up of easily recognizable component peaks. The parts of the chromatograms that occurred at a retention index < 0.15 and at a retention index > 2.8 were removed from the chromatograms, because of unreliable data from these parts, due to noisy mass spectra at the beginning of the chromatograms and column bleeding at the end of the run. The resulting data files were carefully combined. This was done by means of combination of identical compounds from different samples. Compounds were combined based on similarity of mass spectra and retention times. This resulted in a final database file containing almost 6000 different chromatographic peaks. Compound intensities below the detection limit were set at 0. The resulting database was used for statistical analysis.

To determine which compounds were of interest with regard to the classification of CF patients and controls, we used stepwise discriminant analysis, using SPSS (SPSS Inc. Chicago, Illinois, USA). A VOC was only included in the discriminant analysis if its prevalence was 7% or more within the study population, accordingly, 1099 VOCs were included in the analysis [Penn DJ, et al. J R Soc Interface 2007; 4:331-340]. Discriminant analysis was assessed with the grouping variables 'diagnosis' (CF n=48, controls n=57).

Lung function tests were performed after exhaled breath sampling. Bronchodilator medication was stopped prior to lung function testing: short-acting bronchodilators at least 8 hours before, and long-acting bronchodilators at least 36 hours before the test. Dynamic spirometry was performed by means of a pneumotachograph (Flowscreen®, Viasys, Hoechberg, Germany), with measurement of forced expiratory volume in 1 second (FEV1) and forced vital capacity (FVC), according to the standards of the European Respiratory Society [Tammeling GJ, and Quanter PH. Eur Respir J 1993; 6:5-40]. The highest values of FEV1 and FVC of three forced expiratory manoeuvres were used for data analysis. In CF patients, the bronchodilating response was assessed 15 minutes after administration of a short-acting bronchodilator (salbutamol 400 microgram) and expressed as the increase in FEV1 compared to the predicted value of FEV1. In addition, in the CF population, static lung volumes, residual volume (RV), intra-thoracic gas volume (ITGV) and total lung capacity (TLC), were assessed by body plethysmography (Viasys, Hoechberg, Germany).

All data are expressed as mean ± SE. A normal distribution was present for all patient characteristics. Therefore, two-sided independent-sample T-tests were used for the analysis of the subject characteristics. P-values of <0.05 were considered statistically significant. To analyse the VOC profiles, 10 times cross-validation with multiple discriminant analysis was used, to minimize type III errors. Of 10 subsamples, 9 subsamples are used as a training set and the remaining single subsample was used to test the model (validation set). The cross-validation process was repeated 10 times, with each of the 10 subsamples used once as the validation data. The 10 results were averaged to produce a single estimation.

Discriminant analysis was applied in order to: 1) investigate differences among groups, 2) determine the most parsimonious way to distinguish among groups, 3) discard variables which are of little interest related to group distinctions, 4) classify cases into groups, and 5) test theory by observing whether cases are classified as predicted.

### Example 3: Reproducibility and influence of breathing pattern

The influence of the breathing pattern (unforced breathing pattern versus hyperventilation) on VOC profiles, and the variation in VOCs across the day and between days was analysed in 15 healthy control children aged 5-15 yrs (mean ± SD, 15.5 ± 3 yrs). Sampling of VOCs was applied during an unforced breathing pattern (conform the entire study population), during hyperventilation, after one hour, and after one day.

We assumed a minimal sensitivity of at least 70% for a potentially valuable inflammatory marker. The standard error of the sensitivity and the specificity of an inflammatory marker will be less than 5% when 100 children (50 children with CF and 50 controls) are recruited for this study.

As reported previously by van Berkel et al., we did not correct our measurements for chemical background appearing in the samples [van Berkel JJ, et al. J Chromatogr B Analyt Technol Biomed Life Sci 2008; 861:101-107]. This is due to the fact that it will not be possible to correct for the complex interdependencies between excretion and uptake of VOCs by easily subtracting the inhaled from the exhaled air. Moreover, background noise will be randomly distributed between subjects' samples and would thus neither exert any discriminatory power, nor interfere with the outcome of the analyses.

The quantification of the similarity between the VOC profiles was done by means of calculation of a 'distance measure' (dot product rule) or match factor, as been described in detail by Stein et al [Stein SE and Scott DR. Journal of the American Society of Mass Spectrometry 1994; 5:859-866]. This distance measure is based on the similarity of the entire raw chromatogram. A value of '1' denotes identical samples, the lower the value the lesser the degree of similarity. Values of 0.8 and over can be characterised as very good agreement [Stein SE and Scott DR. Journal of the American Society of Mass Spectrometry 1994; 5:859-866].

Distance measure calculation of all complementary files within one hour, resulted in a mean ± SD match factor of 0.90 ± 0.038. Similarity of VOC profiles after one day was 0.85 ± 0.096. These data indicate a low within-subject variation in VOC patterns after one hour, and after a day. Samples were assessed with an unforced breathing pattern and during hyperventilation. Characteristics are shown in Table 3. The match factor (mean ± SD) of these breathing patterns was 0.95 ± 0.043 which indicates minimal influence of breathing pattern on VOC profiles.

**TABLE 3**

| *Characteristics of breathing patterns* | | | | |
|---|---|---|---|---|
| **Attribute** | **Units** | **Standard** | **Hyperventilation** | ***p-value*** |
| Exhalation pressure | mbar | 22.3 ± 11.3 | 64.2 ± 15.5 | < 0.001 |
| Sampling duration | Seconds | 15:45 ± 1:28 | 15 :35 ± 1:16 | 0.11 |
| Number of exhalations | | 3.5 ± 1.6 | 3.9 ± 1.3 | 0.27 |
| Enviromental temperature | ° C | 23.5 ± 0.4 | 23.7 ± 0.6 | 0.30 |
| Enviromental humidity | % | 51.7 ± 9.5 | 50.5 ± 8.1 | 0.27 |

| | | | | |
|---|---|---|---|---|
| *Data are given as mean* ± *SD* | | | | |

## Claims

1. In vitro method of diagnosing cystic fibrosis in a patient by determining the amount of at least one volatile organic compound in a sample derived from a patient suspected of having cystic fibrosis and comparing said amount with a normal value for said volatile organic compound, wherein a difference between the normal value and the amount of said at least one volatile organic compound is indicative of cystic fibrosis, wherein said volatile organic compound is selected from the group consisting of 3,3-dimethylhex-1-ene, 2-buten-1-ol, N-methyl-2-methylpropylamine, C8H16 hydrocarbon (2-octene, 3-octene), tolualdehyde (o-, m-, or p- isomers), C16 poly-unsaturated hydrocarbon, C12 saturated hydrocarbon, C13 saturated hydrocarbon, benzothiazole and long chain alkylbenzenes.

2. Method according to claim 1 wherein said method comprises the step of determining at least the amount of 3,3-dimethylhex-1-ene in said sample.

3. Method according to claim 2 wherein said method consists of the step of determining at least the amount of 3,3-dimethylhex-1-ene in said sample

4. Method according to claims 1 or 2 wherein at least 2 volatile organic compounds are detected.

5. Method according to claim 4 wherein at least 3 volatile organic compounds are detected.

6. Method according to claim 5 wherein at least 4 volatile organic compounds are detected.

7. Method according to claim 6 wherein at least 5 volatile organic compounds are detected.

8. Method according to claim 7 wherein at least 6 volatile organic compounds are detected.

9. Method according to claim 8 wherein at least 7 volatile organic compounds are detected.

10. Method according to claim 9 wherein at least 8 volatile organic compounds are detected.

11. Method according to claim 10 wherein at least 9 volatile organic compounds are detected.

12. Method according to claim 11 wherein at least 10 volatile organic compounds are detected.
